(19) Europäisches Patentamt
European Patent Office
Office européen des brevets

(11) Publication number: **0 186 950**
**B1**

(12) **EUROPEAN PATENT SPECIFICATION**

(45) Date of publication of patent specification: **25.04.90**

(51) Int. Cl.[5]: **C 07 C 231/10**

(21) Application number: **85307982.0**

(22) Date of filing: **04.11.85**

(54) **1,6-Hexanediamides from 3-pentenamides.**

(30) Priority: **29.11.84 US 676364**
**24.12.84 US 685656**
**03.05.85 US 730219**

(43) Date of publication of application:
**09.07.86 Bulletin 86/28**

(45) Publication of the grant of the patent:
**25.04.90 Bulletin 90/17**

(84) Designated Contracting States:
**DE FR GB IT NL**

(56) References cited:
**EP-A-0 080 957**
**EP-A-0 143 303**
**US-A-2 542 766**

**CHEMICAL ABSTRACTS, vol. 69, no. 9, 26th August 1968, page 3300, no. 35491n, Columbus, Ohio, US; & SU - A - 210 138 (ALL-UNION SCIENTIFIC-RESEARCH INSTITUTE OF PETROCHEMICAL PROCESSES) 06-02-1968**

(73) Proprietor: **THE STANDARD OIL COMPANY**
**Midland Building**
**Cleveland, Ohio 44115 (US)**

(72) Inventor: **Cesa, Mark Clark**
**4617 Birchwold Road**
**South Euclid Ohio 44121 (US)**
Inventor: **Burrington, James David**
**23860 Harms Road**
**Richmond Heights Ohio 44143 (US)**
Inventor: **Dubbert, Robert Allen**
**6987 Kingswood Drive**
**Solon Ohio 44139 (US)**

(74) Representative: **Smith, Sydney et al**
**Elkington and Fife Beacon House 113 Kingsway**
**London WC2B 6PP (GB)**

(56) References cited:
**BULLETIN OF THE ACADEMY OF SCIENCES OF THE USSR, DIVISION OF CHEMICAL SCIENCE, vol. 24, no. 8, August 1975, pages 1673-1676, Plenum Publishing Corp., New York, US; B.K. NEFEDOV et al.: "Carbonylation reactions communication 20. Synthesis of n-substituted propionamides from carbon monoxide, ethylene, and amines in presence of some transition-metal salts"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 186 950 B1

**Description**

This invention relates to the production of 1,6-hexanediamides.

In an important aspect this invention relates to a new method of making adipamide or derivatives thereof by the hydrocarbamylation of 3-pentenamide or derivatives thereof.

Adipamide is currently prepared from adiponitrile by controlled hydrolysis. The adiponitrile for the hydrolysis is prepared commercially by electrohydrodimerization of acrylonitrile or by hydrocyanation of 1,3-butadiene.

In US—A—2,542,766, there is described a process for preparing organic nitrogen compounds in which carbon monoxide and ammonia or substituted ammonias are reacted with organic compounds containing olefinic unsaturation to produce compounds containing amido or cyano nitrogen. The general method of preparation disclosed is represented by the following equation

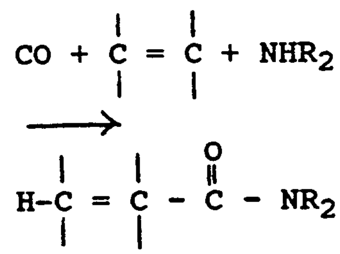

wherein R represents hydrogen or an organic radical.

In Chem. Abstr. *69* (1968), 35491n the synthesis of diamides is disclosed and it is said that to increase the yield of diamides they are prepared from the reaction of diolefins with CO and an amine while heating to 210°C at 250 kg/cm$^2$ in the presence of metal carbonyls and $C_5H_5N$ as a solvent.

It is an object of the present invention to provide a new and improved method of making adipamide or derivatives thereof.

It is a further object of the invention to provide a relatively simplified method of making adipamide from 3-pentenamide.

Other objects, as well as aspects, features and advantages, of the invention will become apparent from a study of the specification, including the claims.

The foregoing and other objects are accomplished by the present invention according to which there is provided a process for making a diamide of the formula

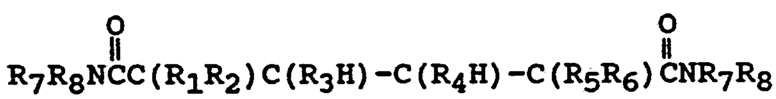

by the catalytic reaction of a monoamide compound of the formula

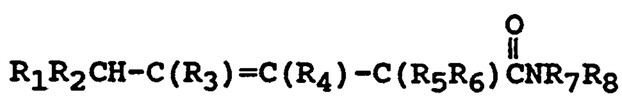

with CO and an amine or ammonia of the formula $R_7R_8NH$ in the liquid phase under essentially anhydrous conditions at a temperture in the range from 120 to 210°C, and a pressure in the range 3,447 to 68,950 kPag (500 to 10,000 psig) where each of $R_1$, $R_2$, $R_3$, $R_4$, $R_5$, $R_6$, $R_7$ and $R_8$ is selected independently from H and a $C_1$ to $C_{12}$ hydrocarbyl containing no ethylenic or acetylenic unsaturation.

In the usual practice of the invention the compound $R_7R_8NH$ is $NH_3$. Especially useful starting material amides are the 3-alkene amides, i.e., where $R_1$, $R_2$, $R_3$, $R_4$, $R_5$ and $R_6$ are all alkyl groups or H, particularly those 3-alkene amides having 5 to 17 carbon atoms, and in particular 3-pentenamide.

The CO pressure used at reaction temperature is in the range from 3,447 to 16,950 kPag (500 to 10,000 psig). Usual reaction times are from 5 to 50 hours.

We believe that the reaction of a substituted or unsubstituted 3-pentenamide with CO and ammonia or a primary or a secondary amine to make a substituted or unsubstituted 1,6-hexanediamides is a reaction entirely undisclosed in the prior art, with or without a catalyst, and we believe that this reaction *per se* is new and unobvious, broadly. In carrying out this reaction, however, we have found a catalyst to be necessary.

One class of such catalysts has the formula

$$M_m(CO)_nL_1X_x$$

where M is one or more of a Group VIII metal, Mn and Re; L is one or more of $R_3P$, $R_2S$, $R_2Se$, $R_3N$, $R_3As$,

$R_3Bi$, $R_3Sb$, $R_2Sn$ and $R_4Sn$, where R is selected from H, or alkyl, aryl, alkoxy, aryloxy, dialkylamido, diarylamido, alkysulfido, and arylsulfido containing 1—30 carbon atoms; X is one or more of F, Cl, Br, I, H, $SnF_3$, $SnCl_3$, $SnBr_3$, SCN, NC, NCS, O, S, Se, $SO_4$, $NO_3$, $PO_4$, $SO_3$, $ClO_4$, $HCO_3$ and $CO_3$; m = 1—16, n = 0—40, 1 = 0—40, and x = 0—16, where $n + 1 + x \geqq m$.

In a representative example, a glass-lined stainless steel high pressure bomb reactor containing a magnetic stir bar was charged with 7.28 mole parts 3-pentenamide, 0.271 mole parts $Co_2(CO)_8$, 3.08 mole parts 4-methlypyridine, 2.8 mole parts triphenylphosphine and 52.9 mole parts $NH_3$, and 0.2 mole parts hexadecane internal standard in 195 mole parts of tetrahydrofuran solvent. Then CO was added to a pressure of 15,169 kPag (2200 psig), and the bomb was sealed and the reaction mixture stirred at 200°C for 18 hours, then cooled to room temperature. The pressure was then released and the reaction mixture was analyzed by gas chromatography. Conversion of 3-pentenamide was 100 percent. Yield of adipamide was 2.6 percent and the yield of 5-methylpyrrolidone was 9.28 percent.

The products of the invention are useful to make the corresponding diamines by known methods, and such diamines are useful to make nylon polyamide high polymer plastics well known methods.

## Claims

1. A process for making a diamide of the formula

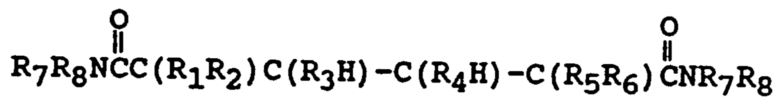

by the catalytic reaction of a monoamide compound of the formula

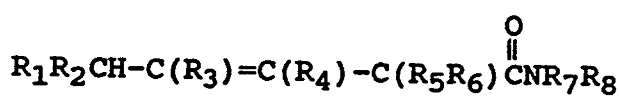

with CO and an amine or ammonia of the formula $R_7R_8NH$ in the liquid phase under essentially anhydrous conditions at a temperature in the range from 120 to 210°C, and a pressure in the range 3,447 to 68,950 kPag (500 to 10,000 psig) where each of $R_1$, $R_2$, $R_3$, $R_4$, $R_5$, $R_6$, $R_7$ and $R_8$ is selected independently from H and a $C_1$ to $C_{12}$ hydrocarbyl containing no ethylenic or acetylenic unsaturation.

2. A process of claim 1 where $R_7$ and $R_8$ are H.

3. A process of claim 1 where $R_7$ and $R_8$ are H and the monoamide is 3-pentenamide.

4. A process of claim 1 where $R_7$ and $R_8$ are H and each of $R_1$, $R_2$, $R_3$, $R_4$, $R_5$ and $R_6$ is an alkyl group or H.

5. A process of claim 4 wherein the said monoamide has 5—17 carbon atoms.

## Patentansprüche

1. Verfahren zur Herstellung eines Diamids der Formel

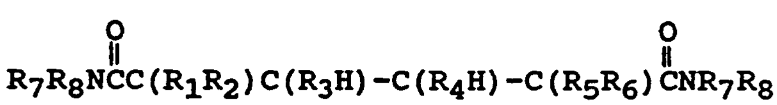

durch katalytische Reaktion einer Monoamid-Verbindung der Formel

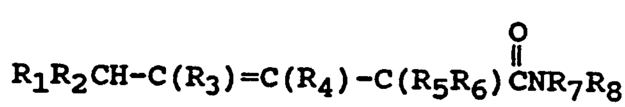

mit CO und einem Amin oder Ammoniak der Formel $R_7R_8NH$, in der flüssigen Phase, unter im wesentlichen wasserfreien Bedingungen, bei einer Temperatur im Bereich von 120 bis 210°C und einem Druck im Bereich von 3447 bis 68950 kPag (500 bis 10000 psig), worin $R_1$, $R_2$, $R_3$, $R_4$, $R_5$, $R_6$, $R_7$ und $R_8$ jeweils unabhängig voneinander aus H und einem $C_1$- bis $C_{12}$-Kohlenwasserstoffrest, der keine ethylenische oder acetylenische Unsättigung enthält, ausgewählt werden.

2. Verfahren nach Anspruch 1, worin $R_7$ und $R_8$ H sind.

3. Verfahren nach Anspruch 1, worin $R_7$ und $R_8$ H sind und das Monoamid 3-Pentenamid ist.

4. Verfahren nach Anspruch 1, worin $R_7$ und $R_8$ H sind und $R_1$, $R_2$, $R_3$, $R_4$, $R_5$ und $R_6$ jeweils eine Alkylgruppe oder H sind.

5. Verfahren nach Anspruch 4, worin das Monoamid 5—17 Kohlenstoffatome aufweist.

**Revendications**

1. Procédé de préparation d'un diamide de formule

$$R_7R_8N\overset{\overset{O}{\|}}{C}C(R_1R_2)C(R_3H)-C(R_4H)-C(R_5R_6)\overset{\overset{O}{\|}}{C}NR_7R_8$$

par réaction catalytique d'un composé formé d'un monoamide de formule:

$$R_1R_2CH-C(R_3)=C(R_4)-C(R_5R_6)\overset{\overset{O}{\|}}{C}NR_7R_8$$

avec CO et une amine ou de l'ammoniac de formule $R_7R_8NH$, en phase liquide et dans des conditions essentiellement anhydres, à une température comprise entre 120 et 210°C et sous une pression relative comprise entre 3 447 et 68 950 kPa (500 à 10 000 livres par pouce carré), chacun des $R_1$, $R_2$, $R_3$, $R_4$, $R_5$, $R_6$, $R_7$ et $R_8$ étant choisi d'une manière indépendante parmi H et un hydrocarbure en $C_1$ à $C_{12}$ ne contenant pas d'insaturation éthylénique ou acétylénique.

2. Procédé suivant la revendication 1, selon lequel $R_7$ et $R_8$ sont H.

3. Procédé suivant la revendication 1, selon lequel $R_7$ et $R_8$ sont H et le monoamide est un pentènamide-3.

4. Procédé suivant la revendication 1, selon lequel $R_7$ et $R_8$ sont H et chacun des $R_1$, $R_2$, $R_3$, $R_4$, $R_5$ et $R_6$ est un radical alcoyle ou H.

5. Procédé suivant la revendication 4, selon lequel le monoamide comprend 5 à 17 atomes de carbone.